# EUROPEAN PATENT SPECIFICATION

(11) **EP 1 257 261 B1**
(45) Date of publication and mention of the grant of the patent: **17.11.2004**
(21) Application number: 00952070.1
(22) Date of filing: 09.08.2000
(51) Int. Cl.: A61K 45/06, A61P 31/18

(54) **ANTIVIRAL PHARMACEUTICAL COMPOSITIONS CONTAINING IRON CHELATORS**
EISENCHELATOR ENTHALTENDE ANTIVIRALE PHARMAZEUTISCHE ZUSAMMENSETZUNGEN
COMPOSITIONS PHARMACEUTIQUES ANTIVIRALES CONTENTANT UN CHELATEUR DE FER

(30) Priority: 13.08.1999 NL 1012825
(43) Date of publication of application: 20.11.2002
(73) Proprietor: Faculteit Geneeskunde Universiteit Utrecht, 3508 TA Utrecht (NL)
(72) Inventor: VAN ASBECK, Bernt, Sweder, NL-3971 RD Driebergen (NL); MARX, Johannes, Josephus, Maria, NL-3584 ZT Utrecht (NL)
(74) Representative: Kupecz, Arpad
(86) International application number: PCT/NL2000/000559
(87) International publication number: WO 2001/012168

(56) References cited:
- WO-A-92/15329
- WO-A-92/16200
- MALLEY S D ET AL: "SYNERGISTIC ANTI-HUMAN IMMUNODEFICIENCY VIRUS TYPE 1 EFFECT OF HYDROXAMATE COMPOUNDS WITH 2',3'-DIDEOXYINOSINE IN INFECTED RESTING HUMAN LYMPHOCYTES" PROCEEDINGS OF THE NATIONAL ACADEMY OF SCIENCES OF USA, NATIONAL ACADEMY OF SCIENCE. WASHINGTON, US, vol. 91, 1 November 1994 (1994-11-01), pages 11017-11021, XP000572692 ISSN: 0027-8424
- GAO W-Y ET AL: "ANTI-HUMAN IMMUNODEFICIENCY VIRUS TYPE 1 ACTIVITY OF HYDROXYUREA INCOMBINATION WITH 2',3'-DIDEOXYNUCLEOSIDES" MOLECULAR PHARMACOLOGY, BALTIMORE, MD, US, vol. 46, no. 4, 1 October 1994 (1994-10-01), pages 767-772, XP000573476 ISSN: 0026-895X
- GEORGIOU NIKI A ET AL: "Inhibition of human immunodeficiency virus type 1 replication in human mononuclear blood cells by the iron chelators deferoxamine, deferiprone, and bleomycin." JOURNAL OF INFECTIOUS DISEASES, vol. 181, no. 2, February 2000 (2000-02), pages 484-490, XP001027530 ISSN: 0022-1899

## Description

The present invention relates to a pharmaceutical product for the treatment of viral infections, in particular of the human immunodeficiency virus (HIV).

Viral infections, in particular infections by the human immunodeficiency virus (HIV) are the serious threat to public health. World-wide much effort goes into the development of a pharmaceutical product to combat this usually incurable disease.

It is known that iron is involved in the replication of human immunodeficiency virus. In the first place, the HIV-1 long terminal repeat (LTR) portion contains nuclear factor (NF)-kB response elements that regulate proviral transcription. NF-kB activation may be influenced with iron via the production of reactive oxygen species.

A second route by which iron chelation can influence HIV replication is by inhibition of the DNA synthesis by inactivation of iron-dependent ribonucleotide reductase.

Another strategy which can be employed in targeting iron chelators against HIV-1 is a direct viral DNA/RNA attack.

Malley et al., 1994 (Proc. Natl. Acad. Sci. USA, Vol. 91 pp. 11017-11021) and Gao et al; 1994 (Molecular Pharmacology, 48, 767-772) disclose the effects of hydroxyurea, an iron chelator, and 2',3'-di-deoxyinosine on HIV-1 replication.

WO 92/15329 discloses a method for inhibiting HIV, employing a composition containing desferrioxamine in conjunction with antiviral compounds such as ddI.

It is the object of the present invention to provide a pharmaceutical product for the efficient treatment of viral infection, in particular of the human immunodeficiency virus, to which end the pharmaceutical product is characterized in that the pharmaceutical product comprises a compound that as active component contains an iron chelator, selected from the family of the hydroxypyridinons (such as deferiprone), and the nucleic acid-binding chemotherapeutical products (such as bleomycin), and another virus-inhibiting compound, which is a protease inhibitor. In particular ritonavir, has been proved to be especially suitable as the antiviral agent.

Another antiviral agent producing a favourable synergistic effect with iron chelators is reverse transcriptase inhibitor, in particular dideoxyinosine.

In this connection applicant has examined the effect of deferoxamine (DF), deferiprone (L1) and bleomycin (BLM) on HIV-1 replication in primary blood cells. For this purpose human peripheral blood lymphocytes (PBL) and monocyte-derived macrophages (MDM), the main target cells for HIV, were used. It was shown, that the considerable production of HIV-1 replication by DF was accompanied by a proliferation inhibition. The same was observed with L1. Surprisingly, BLM inhibited the viral replication in PBL and MDM without affecting lymphocyte proliferation. The use of iron chelators in combination with established antiviral agents for antiviral combination therapy was examined. When DF was used in combination with the nucleoside analog dideoxyinosine (ddI) (not part of the claimed invention), a surprising synergistic inhibition of p24 in HIV-infected PBL seemed to occur.

### Materials and methods used in the study are the following.

### Cell isolation.

Peripheral Blood Mononuclear Cell (PBMC) fractions were isolated from heparinized blood from HIV-1-, HIV-2- and hepatitis B-seronegative donors (Bloodbank, Utrecht, The Netherlands) by Ficoll-Isopaque gradient separation. Cells were washed twice and were then either subjected to counter-current centrifugal elutriation, or monocytes in the PBMC fraction were allowed to adhere to fibronectin-coated flasks before the PBL fraction was harvested. Monocytes were of >95% purity by criteria of cell morphology on May-Grunwald-Giemsa-stained cytosmears and by non-specific esterase staining using (α-naphthylacetate (Sigma Chemical Co., St. Louis, MO) and PBL >85% purity by May-Grunwald-Giemsa-staining. Viability of both cell types was >95% at the point of experiment initiation as determined by trypan-blue exclusion. Isolated monocytes were cultured in suspension at a concentration of 2 x 10⁶ cells/ml in teflon flasks minimizing adherence (Nalgene, Rochester, NY) and allowed to differentiate for 7 days. MDM medium consisted of Dulbecco's modified Eagle's medium supplemented with 10% heat-inactivated human AB serum, negative for anti-HIV antibodies (Bloodbank Utrecht, NL), 10 µg/ml gentamycin (Life Technologies Ltd, Paisley, Scotland), 5 µg/ml ciprofloxacin (Beyer, Leverkusen, Germany), 0.6 mg/ml L-glutamine (Sigma) and 3.4 g/L bicarbonate (Merck, Darmstadt, Germany). Isolated PBL was stimulated to proliferate for 3 days with 4 µg/ml phytohemagglutinin (PHA; Sigma) in RPMI-1640 (Life Technologies Ltd.) medium supplemented with 10% heat inactivated fetal calf serum (Life Technologies Ltd.) and 10 µg/ml gentamycin. After PHA stimulation the PBL were cultured in medium containing 10 U/ml human recombinant IL-2 (Boehringer, Mannheim, Germany). All incubations were carried out in flat-bottomed 96-well plates at 37 °C, 5% CO₂ and 95% air.

### HIV infection and p24 measurements.

Cells were infected for 2 hours with HIV-1_{Ba-L}. Macrophages were infected with a multiplicity of infection (MOI) of 0.005 and lymphocytes with an MOI of 0.001. The cells were then washed twice to remove excess virus and subsequently incubated with either DF (Novartis Pharma, Arnhem, The Netherlands), L1 (from Duchefa Farma B.V., Haarlem, The Netherlands) or BLM (H. Lundbeck A/S, Copenhagen, Denmark) for a period of 5 days. Virus in culture supernatant was inactivated in a final concentration of 0.05% empigen (Calbiochem-Novabiochem Co., La Jolla, CA). The presence of HIV-1 in the inactivated supernatant was monitored by checking for the p24-core antigen by enzyme-linked immunosorbent assay (ELISA).

### Lymphocyte proliferation measurements.

Since iron chelators may influence cellular proliferation the effect of the three iron chelators on PBL proliferation was also determined. This was performed by monitoring overnight ³H-thymidine (³H-TdR) incorporation into DNA. 25 µl ³H-TdR (0.5 µCi, Amersham International plc, UK) was added in the presence of DF, L1, BLM or DF/ddI combination, in a total volume of 200 µl cell suspension after 4 days incubation with the different compound concentrations. Cell incubations were in 96-well plastic plates (Costrar, Cambridge, MA, US). After overnight incubation with ³H-TdR, the cells were harvested onto a filter using an automatic cell microharvester (Titertek® cell harvester 530). The filter was subsequently dried and immersed in a scintillation cocktail (Wallac UK). Radioactivity was counted in a Mark II liquid Scintillation system (Model x, Tricton analytics, Inc.).

### Cytotoxicity.

Possible cytotoxicity of DF, L1 or BLM was monitored by the MTT [3-(4,5 dimethylthiazol-2-yl)-2,5-diphenyltetrazolium bromide] (Sigma) assay.

### Studies of DF-ddI combination (Not part of the claimed invention).

Three separate experiments were performed substantially as described in the section *HIV infection and p24 measurements.* Multiply-diluted fixed-ratio combinations of the drugs, or single drugs were added to each incubation point. The IC₅₀ (inhibitory concentration) of each drug was calculated using the computer software program CalcuSyn. The combined-drug effect was evaluated by the median-effect principle and the isobologram method. This method involves the plotting of the dose-effect curve for each drug alone and in combination. The slope of the median-effect plot and the *x* intercept of the plot were then used for the computerized calculation of a combination index (CI). The CI values were based on the classic isobologram equation and CI values of <1, 1 and >1 indicate synergism (greater than the expected additive effect), additive effects and antagonism (less than the expected additive effect). Fraction affected (fₐ) was calculated for every drug combination and for each drug alone after p24 measurements in infected PBL incubated for 5 days with the examined compounds (see *HIV infection and p24 measurements*).

### Statistical analysis.

Repeated measures ANOVA and student Newman-Keuls test were used to analyze the data. *P* values below 0.05 were considered significant.

### Results

### Effect of DF on HIV-1 replication, cellular proliferation and cytotoxicity in PBL.

DF was shown to reduce p24 levels in culture supernatants. At a concentration of 3 µM DF a 40% reduction was observed in viral replication in PBL (19 vs 11 ng p24/ml, n=3, p<0.05). A compound concentration of 30 µM reduced p24 levels by >90% (figure IA) from 19 to 1.9 ng p24/ml (n=3). To rule out the possibility that these inhibitory effects were due to cytotoxicity, cell viability was monitored. A DF concentration of 100 µM was found to be cytotoxic (figure IB). In addition, due to the anti-proliferative properties of DF, cellular proliferation was monitored. The observed reduction in p24 was mirrored by a decrease in proliferation (figure IC). Thus, non-cytotoxic DF concentrations were capable of reducing HIV replication and this reduction was accompanied by cellular proliferation inhibition.

### Effect of L1 on HIV-1 replication, cellular proliferation and cytoloxicity in PBL.

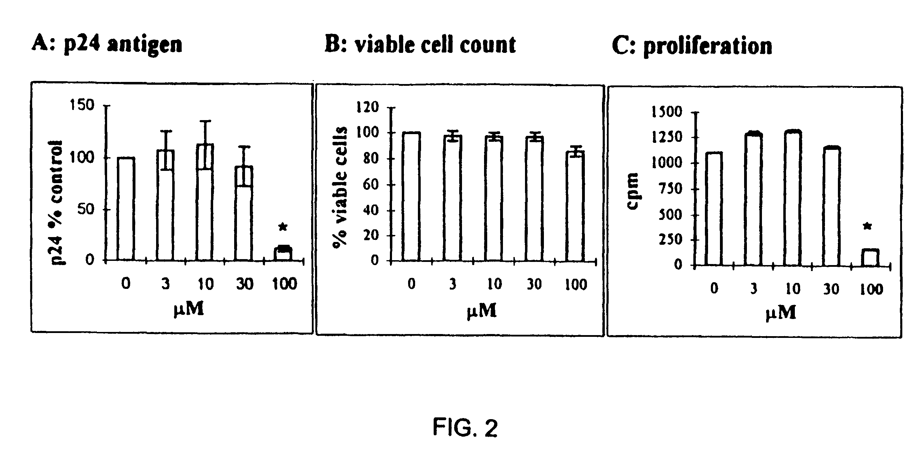

HIV-1 replication in lymphocytes was reduced to minimal levels by 100 µM L1 (figure 2A). This concentration was not cytotoxic in PBL (figure 2B). There seemed to be a threshold point in L1 concentration where the inhibition slope became very steep and this phenomenon was also mirrored by a decrease in proliferation (figure 2C). Thus, the HIV-inhibition observed with L1 could also be attributed to cellular proliferation inhbition. At lower compound concentrations a subtle increase in p24 antigen production was observed (figure 2A).

### Effect of BLM on HIV-1 replication, cellular proliferation and cytotoxicity in PBL.

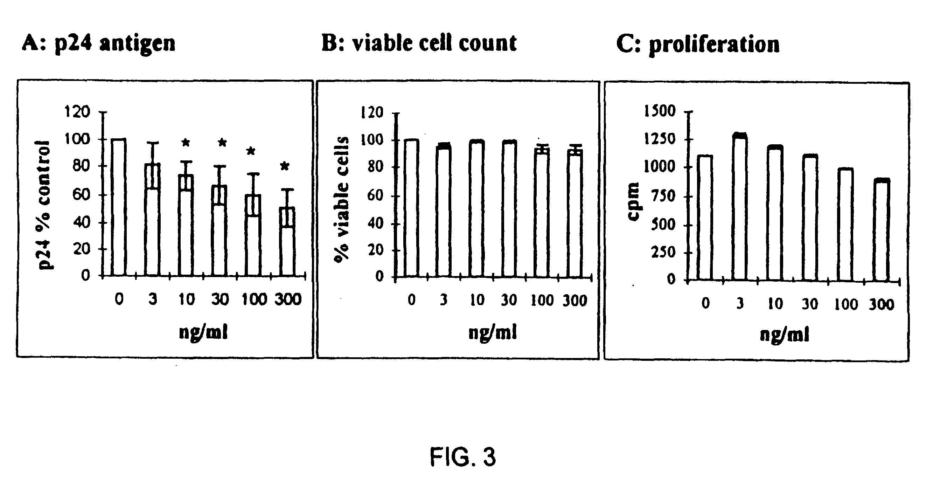

BLM dose-dependently decreased HIV-1 replication in lymphocytes (figure 3A). This compound was not cytotoxic in PBL (figure 3B). At concentrations in the range of 3-300 ng BLM/ml a 20-50% reduction was noted, reflecting a decrease from 19 to 15 (20%) or 9.5 (50%) ng p24/ml (n=3) (figure 3A). Cellular proliferation remained intact (figure 3C). Thus, HIV-1 inhibition by BLM, in contrast to DF and L1, seems to proceed via a pathway other than cellular proliferation inhibition.

### Effect of DF, L1, and BLM on HIV-1 replication and cytotoxicity in MDM.

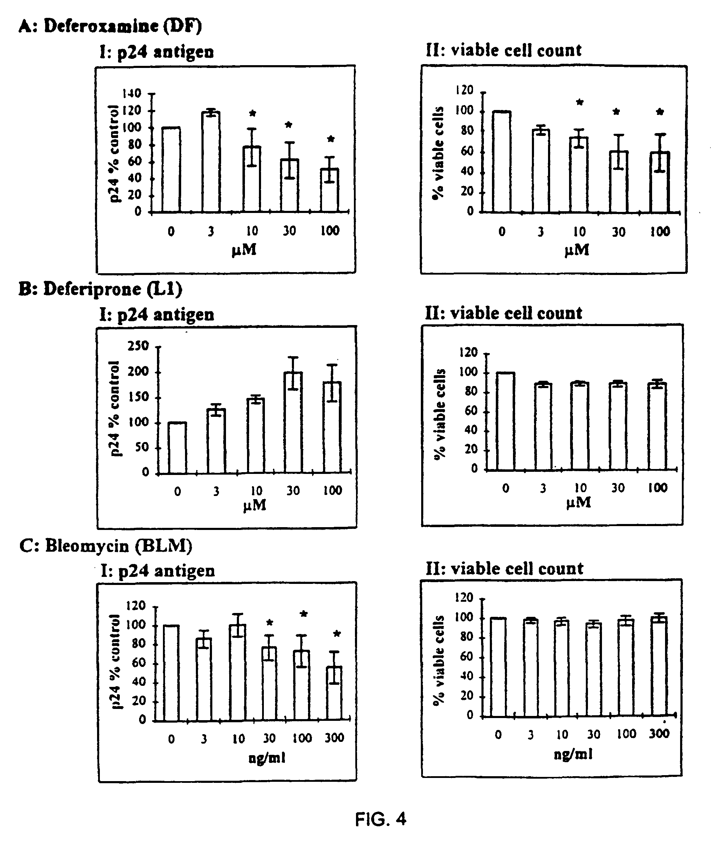

In MDM, DF concentrations around 100 µM reduced viral replication by up to 50% (1.2 vs 0.6 ng p24/ml) in a dose-dependent manner (figure 4AI). However this p24 decrease was associated with the onset of cytotoxicity (figure 4AII). L1 in the concentrations studied, strikingly up-regulated p24 antigen production by up to two fold at 30 µM (figure 4BI). These concentrations were not cytotoxic (figure 4BII).

In MDM a dose-dependent decrease in p24 levels was also observed with a 50% reduction at 300 ng BLM/ml from 1.2 to 0.6 ng p24/ml (figure 4CI). BLM concentrations, similarly to PBL, were found not to affect cell viability in MDM (figure 4CII).

### Combined antiviral effects of DF-ddI (Not part of the claimed invention).

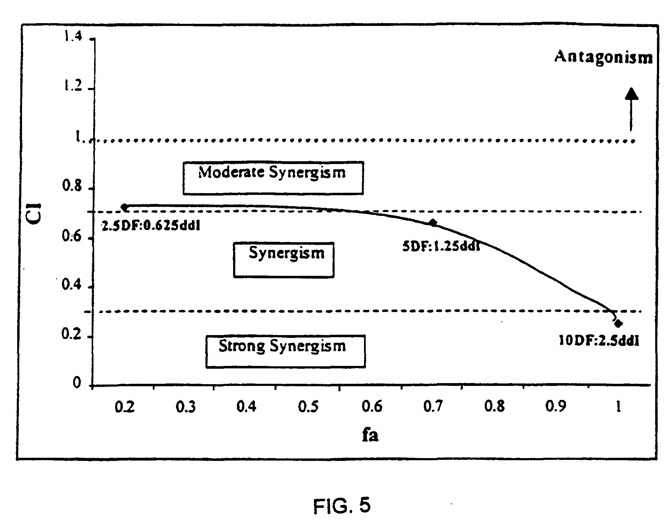

Moderate to strong synergism was detected when DF was used in combination with ddI. Figure 5 shows a representative graph of the combination index (CI) with respect to fraction affected (fₐ) for the inhibition of HIV by a mixture of DF and ddI (molar ratio 4:1). IC₅₀ of DF was 8.5 µM and that of ddI 3.7 µM. Figure 5 clearly shows that there is a marked synergism at all fₐ values obtained with the different combinations, with the synergism getting stronger as fₐ increases. At around the IC₅₀ concentrations of both DF and ddI (combination points on figure 5: 10 µM DF: 2.5 µM ddI), the highest fₐ value was obtained and the combination was strongly synergistic. Cellular proliferation was also monitored with these combinations and with each drug alone. It was found that ddI in combination with DF did not have an additional inhibitory effect on cellular proliferation when compared to DF alone (results not shown).

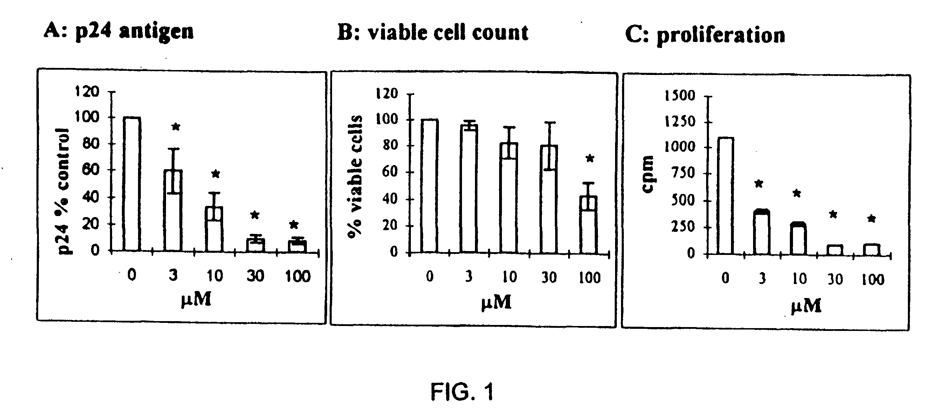

The invention is explained in more detail with reference to the Figures 1-6.

Fig. 1. Effect of DF on HIV-I replication (A), cytotoxicity (B) and cellular proliferation (C) in PBL after 5 days incubation with DF.

For the HIV-1 replication measurements cells were infected for 2 hours with HIV-1_{Ba-L} at an MOI of 0.001; the cells were subsequently washed 2 times and incubated with different DF concentrations. After 5 days, supenatant was harvested for p24 ELISA. Virus replication was expressed as % p24 reduction after DF incubations, compared to p24 measured from control HIV-infected cells incubated in the absence of DF (set at 100% p24). Cytotoxicity was measured by the MTT test and is represented as % viable cells after 5 days DF incubations, compared to viable cells from control HIV-infected cells incubated in the absence of DF (set at 100% viable cells). PBL proliferation was measured by overnight ³H-thymidine incorporation after DF was incubated for 4 days with cells; it is represented as counts per minute (cpm) after DF incubations, compared to cpm from control cells, incubated in the absence of DF. Results are the average of 3 experiments in duplicate (statistical analysis using repeated ANOVA and student Newman-Keuls test, *p<0.05). 100% p24 production represents 19 ng p24/ml

Fig. 2. Effect of L1 on HIV-1 replication (A), cytotoxicity (B) and cellular proliferation (C) in PBL after 5 days incubation with L1.

For the HIV-I replication measurements cells were infected for 2 hours with HIV-1_{Ba-L} at an MOI of 0.001; the cells were subsequently washed 2 times and incubated with different L1 concentrations. After 5 days, supernatant was harvested for p24 ELISA. Virus replication was expressed as % p24 reduction after L1 incubation, compared to p24 measured from control HIV-infected cells incubated in the absence of L1 (set at 100% p24). Cytotoxicity was measured by the MTT test and is represented as % viable cells after 5 days L1 incubations, compared to viable cells from control HIV-infected cells incubated in the absence of L1 (set at 100% viable cells). PBL proliferation was measured by overnight ³H-thymidine incorporation after L1 was incubated for 4 days with cells; it is represented as counts per minute (cpm) after L1 incubations, compared to cpm from control cells, incubated in the absence of L1. Results are the average of 3 experiments in duplicate (statistical analysis using repeated ANOVA and student Newman-Keuls test, *p<0.05). 100% p24 production represents 19 ng p24/ml.

Fig. 3. Effect of BLM on HIV-1 replication (A), cytotoxicity (B) and cellular proliferation (C) in PBL after 5 days incubation with BLM.

For the HIV-1 replication measurements cells were infected for 2 hours with HIV-1_{Ba-L} at an MOI of 0.001; the cells were subsequently washed 2 times and incubated with different BLM concentrations. After 5 days, supernatant was harvested for p24 ELISA. Virus replication was expressed as % p24 reduction after BLM incubations, compared to p24 measured from control HIV-infected cells incubated in the absence of BLM (set at 100% p24). Cytotoxicity was measured by the MTT test and is represented as % viable cells after 5 days BLM incubations, compared to viable cells from control HIV-infected cells incubated in the absence of BLM (set at 100% viable cells). PBL proliferation was measured by overnight ³H-thymidine incorporation after BLM was incubated for 4 days with cells; it is represented as counts per minute (cpm) after BLM incubations, compared to cpm from control cells, incubated in the absence of BLM. Results are the average of 3 experiments in duplicate (statistical analysis using repeated ANOVA and student Newman-Keuls test, *p<0.05). 100% p24 production represents 19 ng p24/ml

Fig. 4. Effect of DF (AI-II), L1 (BI-II) and BLM (CI-II) on HIV-1 replication (A-CI) and cytotoxicity (A-CII) in MDM after 5 days incubation.

For the HIV-1 replication measurements cells were infected for 2 hours with HIV-1_{Ba-L} at an MOI of 0.001; the cells were subsequently washed 2 times and incubated with different compound concentrations. After 5 days supernatant was harvested for p24 ELISA; Virus replication was expressed as % p24 reduction after compound incubations, compared to p24 measured from control HIV-infected cells incubated in the absence of compounds (set at 100% p24). Cytotoxicity was measured by the MTT test and is represented as % viable cells after 5 days compound incubations, compared to viable cells from control HIV-infected cells incubated in the absence of compounds (set at 100% viable cells). Results are the average of 3 experiments in duplicate (statistical analysis using repeated ANOVA and student Newman-Keuls test, *p<0.05). 100% p24 production represents 1.2 ng p24/ml.

Fig. 5. Graphical presentation of the combination index (CI) with respect to fraction affected (fa) for the p24-antigen inhibition by a mixture of DF and ddI (molar ratio 4:1). Data points on the graph are indicated as conc DF and ddI, both in µM. CT < 1, = 1 and > 1 represent synergistic, additive and antagonistic effects, respectively. The subdivision within the synergistic area (CI >0.7, 0.3-0.7 and <0.3 implying moderate synergism, synergism, and strong synergism respectively) is according to Chou and Talalay. The parameters are obtained using the median effect equation of Chou and Talalay and the CalcuSync computer software programme as described in materials and methods. The IC₅₀ within this experiment of DF was 8.5 µM and that of ddI was 3.7 µM. This graph is representative of 3 independent experiments.

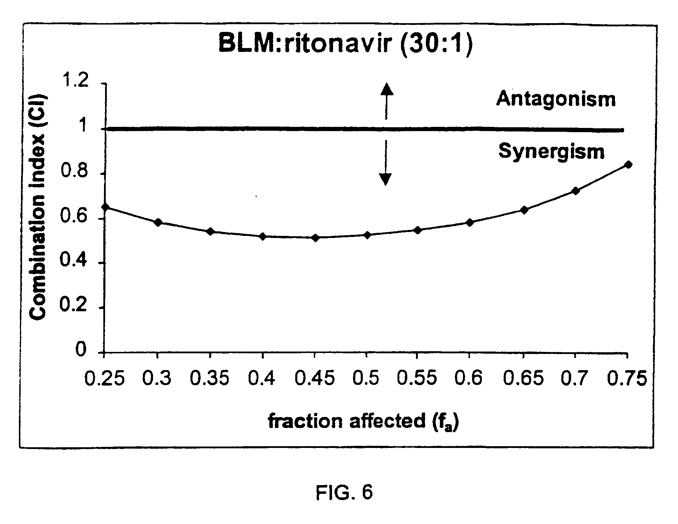

Fig. 6. Graphical presentation of the combination index (CI) with respect to fraction affected (fa) for the p24-antigen inhibition by a mixture of bleomycin (BLM) and ritonavir (ratio 30:1). Peripheral blood lymphocytes (PBL) were infected for 2 hours with HIV-1_{Ba-L} at an MOI of 0.001. The cells were subsequently washed 2 times to remove an excess in virus and subsequently incubated for 5 days with only BLM, only ritonavir or various combinations of the two substances. Virus in the supernatant of the cell cultures was inactivated in a final concentration of 0.05% empigen and the presence of HIV-1 in the inactivated supernatant was measured by determining the p24 antigen with enzyme-linked immunosorbent assay (ELISA). CI<1, = 1 and >1 indicate synergistic, additive and antagonistic effects, respectively, according to Chou and Talalay. The parameters are obtained using the median effect equation of Chou and Talalay, and the CalcuSync computer software programme. In this experiment the IC₅₀ of BLM was 1.9 µg/ml and of ritonavir 0.04 µM. This graph is representative of two independent experiments.

## Claims

1. A pharmaceutical product for the treatment of viral infections, in particular of the human immunodeficiency virus (HIV), **characterized in that** said pharmaceutical product comprises a compound that as active component contains an iron chelator, which is selected from the family of the hydroxypyridinons (such as deferiprone), and the nucleic acid-binding chemotherapeutical products (such as bleomycine), and a component comprising another virus-inhibiting compound, which is a protease-inhibitor.

2. A pharmaceutical product according to claim 1, **characterized in that** the protease-inhibitor is ritonavir.

3. A pharmaceutical product according to claim 1, **characterized in that** the virus-inhibiting compound is a reverse transcriptase inhibitor.

4. A pharmaceutical product according to claim 3, **characterized in that** the reverse transcriptase inhibitor is a dideoxyinosine.

## Patentansprüche

1. Pharmazeutisches Erzeugnis für die Behandlung von Virusinfektionen, insbesondere des human immunodeficiency virus (HIV), **dadurch gekennzeichnet, dass** es eine Verbindung, die als Wirkstoff ein Eisen chelatisierendes Mittel enthält, das aus der Familie der Hydroxypyridinone (wie Deferipron) ausgewählt ist, Nucleinsäure bindende chemotherapeutische Produkte (wie Bleomycin) und eine Komponente, die eine weitere Virus-inhibierende Verbindung umfasst, die ein Protease-Inhibitor ist, umfasst.

2. Pharmazeutisches Erzeugnis nach Anspruch 1, **dadurch gekennzeichnet, dass** der Protease-Inhibitor Ritonavir ist.

3. Pharmazeutisches Erzeugnis nach Anspruch 1, **dadurch gekennzeichnet, dass** die Virus-inhibierende Verbindung ein Reverse-Transkriptase-Inhibitor ist.

4. Pharmazeutisches Erzeugnis nach Anspruch 3, **dadurch gekennzeichnet, dass** der Reverse-Transkriptase-Inhibitor ein Didesoxyinosin ist.

## Revendications

1. Produit pharmaceutique pour le traitement d'infections virales, et en particulier du virus du syndrome immunodéficitaire acquis (SIDA ou HIV), ***caractérisé en ce que*** ledit produit pharmaceutique comprend un composé qui contient comme ingrédient actif un chélateur du fer qui est choisi dans la famille des hydroxypyridinones (telle que la défériprone), et des produits chimiothérapeutiques liant les acides nucléiques (telle la bléomycine), et un composant comprenant un autre composé inhibiteur de virus, qui soit un inhibiteur de protéases.

2. Produit pharmaceutique selon la revendication 1, ***caractérisé en ce que*** l'inhibiteur de protéases est le ritonavir.

3. Produit pharmaceutique selon la revendication 1, ***caractérisé en ce que*** le composé inhibiteur de virus est un inhibiteur de la transcriptase inverse (ou polymérase H).

4. Produit pharmaceutique selon la revendication 3, ***caractérisé en ce que*** l'inhibiteur de la transcriptase inverse est une didanosine (ou didéoxyinosine).
